# FASCICULE DE BREVET EUROPEEN

(11) **EP 0 900 093 B1**
(45) Date de publication et mention de la délivrance du brevet: **08.10.2003**
(21) Numéro de dépôt: 98900962.6
(22) Date de dépôt: 29.01.1998
(51) Int. Cl.: A61M 1/16

(54) **CIRCUIT HYDRAULIQUE POUR LIQUIDE DE DIALYSE**
HYDRAULISCHE SCHALTUNG FÜR DIALYSIERFLÜSSIGKEITEN
HYDRAULIC CIRCUIT FOR DIALYSIS LIQUID

(30) Priorité: 31.01.1997 IT TO970070
(43) Date de publication de la demande: 10.03.1999
(73) Titulaire: GAMBRO HOSPAL (Schweiz) AG, 4001 Basel (CH)
(72) Inventeur: VINCI, Luca, I-46025 Poggio Rusco (IT)
(74) Mandataire: Lejeune, Daniel
(86) Numéro de dépôt international: IB9800117
(87) Numéro de publication internationale: WO98033537

(56) Documents cités:
- EP-A- 0 623 357
- US-A- 3 976 574
- US-A- 4 085 046
- US-A- 5 431 811

## Description

La présente invention concerne un circuit hydraulique pour un liquide de dialyse mis en circulation dans un appareil de dialyse.

Il est connu que les appareils de dialyse comprennent un circuit hydraulique pour l'alimentation en liquide de dialyse frais d'un dialyseur et pour l'évacuation du- liquide usé (liquide de dialyse et ultrafiltrat) sortant du dialyseur. Le circuit de liquide de dialyse est formé par une pluralité de tubes connectés à des moyens de commande et de contrôle (pompe, vannes, transducteurs et appareils de mesure) et à des filtres (dialyseur, ultrafiltre) par l'intermédiaire de raccords et de connexions appropriés. Dans un tel circuit, il importe de maintenir la pression sous contrôle pour éviter que celle-ci ne dépasse une valeur déterminée. En effet, les surpressions éventuelles à l'intérieur du circuit peuvent provoquer une dissociation des tubes et de leurs raccords et par conséquent causer des fuites de liquide et un fonctionnement défectueux de l'appareil.

D'autre part, l'emploi de vannes de sécurité de type connu dans le circuit d'un appareil de dialyse présente quelques inconvénients, liés au matériau des éléments du circuit, à la difficulté d'assurer une stérilisation adéquate pour l'utilisation envisagée, à l'encombrement, à un coût excessif ou à une fiabilité insuffisante.

Le document US 3976574, voir préambule de la revendication 1, concerne une machine de dialyse ayant un circuit hydraulique pour l'alimentation de liquide de dialyse frais vers un dialyseur et pour l'évacuation du liquide usé sortant du dialyseur. Un régulateur de pression est prévu entre la ligne de liquide frais et la ligne de liquide usé.

Le but de l'invention est de réaliser un circuit d'hémodialyse dépourvu des inconvénients mentionnés plus haut.

Pour atteindre ce but, on prévoit, conformément à l'invention, un circuit hydraulique pour liquide de dialyse comprenant une première section de conduite et une deuxième section de conduite, caractérisé en ce qu'il comprend des moyens de dérivation reliant la première section de conduite à la deuxième section de conduite pour dériver une partie du liquide de dialyse circulant dans la première section de conduite vers la deuxième section de conduite quand la pression dans la première section de conduite dépasse une valeur de seuil prédéterminée.

Selon une caractéristique de l'invention, les moyens de dérivation comprennent une vanne comprenant un premier conduit et un deuxième conduit communiquant par un orifice, et des moyens d'obturation montés élastiquement pour fermer (respectivement ouvrir) l'orifice lorsque la pression de liquide dans le premier conduit est inférieure (respectivement supérieure) à une valeur de seuil prédéterminée, le premier conduit étant raccordé à la première section de conduite et le deuxième conduit étant raccordé à la deuxième section de conduite.

D'autres caractéristiques et avantages de l'invention apparaîtront mieux à la lecture de la description qui va suivre. On se reportera aux dessins annexés, sur lesquels:
la figure 1 est un schéma partiel d'une machine de dialyse équipée d'un premier mode de réalisation du circuit d'alimentation en liquide de dialyse selon l'invention;
la figure 2 est une vue en perspective de la vanne de sécurité du circuit de la figure 1;
la figure 3 est une vue en coupe transversale de la vanne de la figure 2; et
les figures 4 et 5 représentent des variantes du circuit de la figure 1.

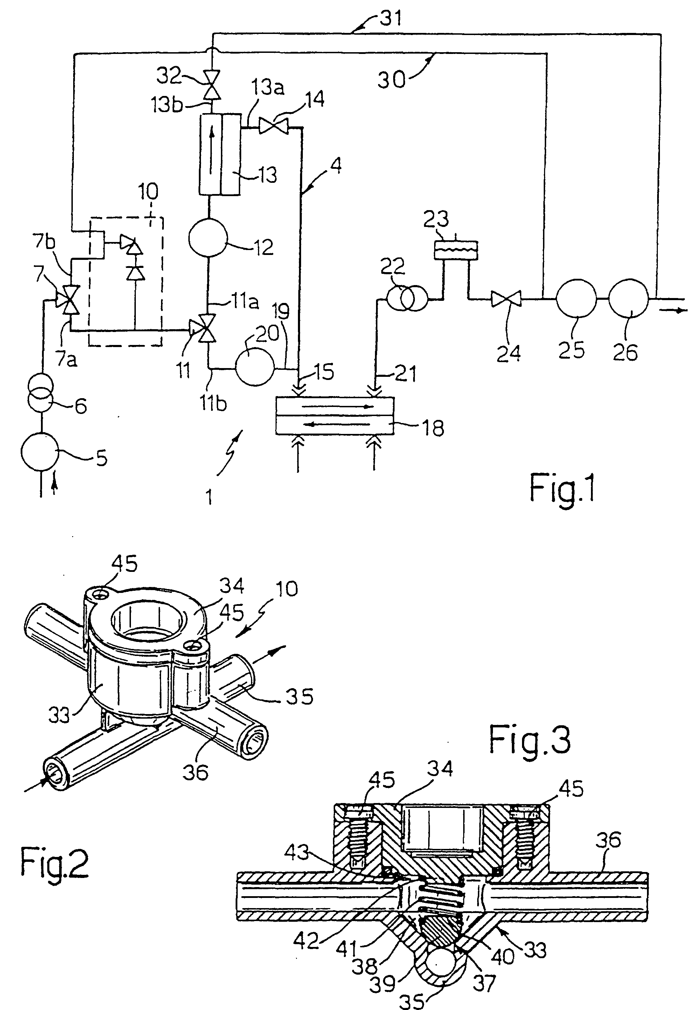

Sur la figure 1, le circuit d'alimentation en liquide de dialyse représenté est repéré par le chiffre de référence 1. Il est représenté partiellement, dans la limite utile à la compréhension de l'invention. En particulier, on n'a pas représenté sur la figure le générateur de liquide de dialyse qui est raccordé au circuit en amont de la partie de circuit représentée (moyens de connexion à une source de liquide extérieure, pompes pour solutions concentrées, moyens de chauffage et de dégazage du liquide de dialyse, etc.)

Le circuit 1 comprend une conduite principale 4 (représentée en traits gras sur la figure) constituée de plusieurs sections, sur laquelle sont disposés, dans le sens de la circulation du liquide, un débitmètre 5, une pompe à engrenages 6 et une électrovanne à trois voies 7. La sortie principale 7a de l'électrovanne 7 est reliée, par l'intermédiaire d'une vanne de sécurité 10 qui sera décrite plus loin en détail, à l'entrée d'une électrovanne 11 ayant une sortie principale 11a reliée, par l'intermédiaire d'un capteur de pression 12, à l'entrée d'un ultrafiltre 13. La sortie principale 13a de l'ultrafiltre 13 est reliée, par l'intermédiaire d'une électrovanne 14, à l'entrée de liquide de dialyse 15 d'un dialyseur 18. Une première conduite de dérivation 19 relie une sortie secondaire 11b de l'électrovanne 11 à l'entrée 15 de liquide de dialyse du dialyseur 18. Un capteur de pression 20 est monté sur la première conduite de dérivation 19.

Sur la section de conduite principale 4 connectée à la sortie 21 de liquide de dialyse du dialyseur 18 sont disposés, dans le sens de circulation du liquide, une pompe à engrenages 22, un dispositif de dégazage 23, une électrovanne 24 de type tout ou rien, un interrupteur de pression 25, et un débitmètre 26. La conduite principale 4 est munie également de moyens d'évacuation de liquide de dialyse usé, non représentés.

Une conduite auxiliaire 30 relie la sortie secondaire 7b de l'électrovanne 7 à la conduite principale 4, en amont de l'interrupteur de pression 25. La conduite auxiliaire 30 est raccordée à la vanne de sécurité 10 (comme cela est décrit ci-dessous). Enfin, une seconde conduite de dérivation 31 sur laquelle est disposée une électrovanne 32 relie une sortie secondaire 13b de l'ultrafiltre 13 à la conduite principale 4, en aval du débitmètre 26.

Comme on peut le voir sur les figures 2 et 3, la vanne de sécurité 10 comprend un corps de vanne 33, par exemple en polypropylène, ayant une cavité interne tronconique 38 à laquelle sont raccordés deux conduits 35, 36 perpendiculaires superposés. Les deux conduits 35, 36 communiquent par l'intermédiaire de la chambre tronconique 38, le conduit supérieur ayant deux tronçons diamétralement opposés débouchant dans la paroi latérale de la chambre tronconique 38 et la chambre tronconique ouvrant par un orifice circulaire 37, à son extrémité de plus petit diamètre, dans le conduit inférieur 35. La chambre tronconique 38 définit, autour de l'orifice circulaire 37, un siège pour un obturateur 39 ayant une extrémité plane opposée à une extrémité arrondie dimensionnée pour pénétrer partiellement dans l'orifice circulaire 37. Le corps de vanne 33 est fermé par un couvercle 34 maintenu en place par deux vis 45. Un ressort hélicoïdal 41 est disposé entre une face interne du couvercle 34 et l'extrémité plane de l'obturateur 39 pour maintenir celui-ci appliqué sur le siège défini par la chambre conique 38. Le ressort 41 est dimensionné pour appliquer une force prédéterminée sur l'obturateur 39 qui conditionne la pression d'ouverture souhaitée de la vanne 10.

Le conduit 35 est raccordé à la conduite principale 4 entre la sortie principale 7a de l'électrovanne 7 et l'entrée de l'électrovanne 11. Le conduit 36 est raccordé à la conduite auxiliaire 30, en aval de l'électrovanne 7.

Le fonctionnement de la machine de dialyse qui vient d'être décrite est le suivant:

Au cours d'une séance de dialyse, dans des conditions de fonctionnement normales, le liquide de dialyse circule dans la conduite principale 4 et traverse tous les dispositifs qui y sont disposés, en particulier les débitmètres 5 et 26, les pompes 6 et 22, l'utrafiltre 13, le dialyseur 18, les vannes 7, 10, 11, 24. Dans la vanne 10 la pression du liquide de dialyse est inférieure à la force exercée sur l'obturateur 39 par le ressort 41. En conséquence, la vanne 10 reste fermée et tout le liquide de dialyse s'écoule dans le conduit 35, c'est-à-dire aussi dans la conduite principale 4.

Si, au cours d'une séance de traitement, la pression du liquide de dialyse augmente jusqu'à surmonter la force du ressort 41, la vanne 10 s'ouvre et une partie du liquide de dialyse s'écoule dans le conduit 36 et dans la conduite auxiliaire 30. La pression dans la conduite principale 4 diminue alors immédiatement et aucun risque de fuite n'est à craindre. La vanne 10 se comporte comme une vanne de limitation de pression et elle n'influe pas sur les conditions normales de fonctionnement du circuit. En effet, comme la conduite auxiliaire 30 se raccorde à la conduite principale 4 en amont du débitmètre 26, l'évacuation de liquide par la vanne de sécurité 10 en cas de surpression ne perturbe pas la comparaison des débits d'entrée et de sortie de liquide dans le circuit, sur laquelle repose la mesure du débit d'ultrafiltration lorsque celle ci est effectuée selon la méthode exposée dans le brevet EP O 243 284.

Pour étalonner les débitmètres 5 et 6, ceux-ci sont mis directement en série. Pour ce faire, la sortie secondaire 7b de l'électrovanne 7 est activée et le liquide mis en circulation par la pompe 6 s'écoule exclusivement dans le conduit 36 de la vanne 10 et dans la conduite auxiliaire 30.

Pour entretenir (désinfection, nettoyage, détartrage) le circuit 1, un liquide d'entretien est mis en circulation dans la conduite principale 4 (par la sortie principale 7a de l'électrovanne 7, qui est reliée au conduit 35 de la vanne 10), puis dans la conduite auxiliaire 30 (par la sortie secondaire 7b de l'électrovanne 7, qui est reliée au conduit 36 de la vanne 10). Comme les conduits 35 et 36 ne sont séparés, en position de fermeture de la vanne 10, que par la surface de contact entre l'obturateur 39 et la paroi de la chambre tronconique 38, cette procédure d'entretien permet de laver complètement la vanne 10 avec le liquide d'entretien, sans qu'il ne soit nécessaire de créer une surpression dans la conduite principale 4 pour faire passer du liquide d'entretien du conduit 35 vers le conduit 36.

Au début de chaque dialyse, il est possible de vérifier l'étanchéité de la vanne 10 en provoquant une surpression dans la conduite principale 4 contrôlée par le capteur de pression 12; en outre, il est possible de vérifier également le fonctionnement correct de la vanne 10 en augmentant la pression du liquide circulant dans la conduite principale 4 jusqu'à ce qu'il se produise une chute brutale de pression au moment de l'ouverture de la vanne 10. Si cette chute de pression se produit juste après que la pression dans la conduite 4, telle que mesurée par le capteur de pression 12, a atteint la valeur de tarage de l'obturateur 39, c'est que la vanne 10 fonctionne correctement.

Le circuit qui vient d'être décrit présente les avantages suivants: l'utilisation de la vanne 10 permet d'éviter l'endommagement du circuit (fuites) causé par les surpressions; la simplicité de la vanne garantit la fiabilité du circuit; la possibilité de raccorder la vanne 10 à des conduites d'un circuit existant permet d'améliorer la sécurité de fonctionnement d'un circuit existant sans entraîner de dépenses considérables; comme les conduits 35 et 36 sont séparés par une zone très réduite et qu'il n'existe pas dans la vanne 10 de zone de stagnation de liquide, la vanne peut être complètement lavée facilement; la vanne définit un trajet d'écoulement unidirectionnel qui ne permet le passage de liquide que de la conduite principale 4 (par l'intermédiaire du conduit 35) vers la conduite auxiliaire 30 (par l'intermédiaire du conduit 36).

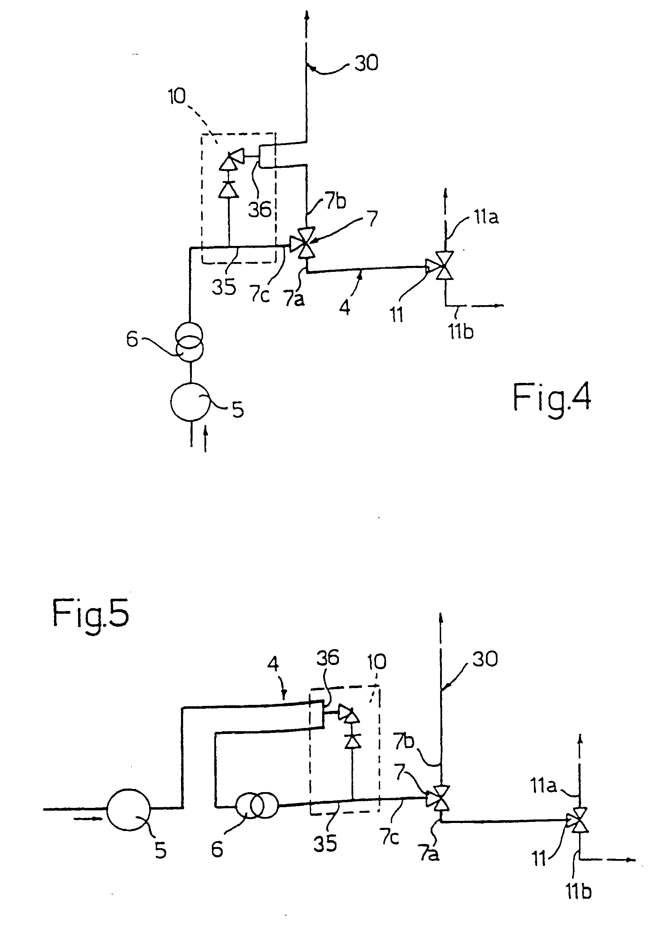

La figure 4 représente une variante du circuit d'alimentation 1 où la vanne de sécurité 10 est disposée en amont de l'électrovanne 7, entre l'entrée 7c de cette électrovanne 7 et la pompe 6. De façon plus détaillée, le conduit 35 de la vanne 10 est raccordé à la conduite principale 4 entre la pompe 6 et l'entrée 7c de l'électrovanne 7, et le conduit 36 est raccordé à la conduite auxiliaire 30 en aval de la sortie secondaire 7b de l'électrovanne 7.

Dans ce circuit lorsque la pression en aval de la pompe 6, par exemple en raison d'un défaut de fonctionnement de celle-ci, atteint la valeur de seuil à laquelle l'ouverture de la vanne 10 est réglée, la vanne 10 s'ouvre et une partie du liquide de dialyse est évacuée par la conduite auxiliaire 30 (sans passer au travers de l'électrovanne 7, au contraire de ce que l'on observe dans le circuit de la figure 1).

La figure 5 représente une autre variante du circuit d'alimentation 1 où la vanne de sécurité 10 est aussi disposée en amont de l'électrovanne 7. Mais à la différence du circuit de la figure 4, dans cette variante, la vanne 10 se décharge dans la conduite principale 4 en cas de surpression. De façon plus détaillée, le conduit 35 est raccordé à la conduite principale 4 entre la pompe 6 et l'entrée 7c de l'électrovanne 7, et le conduit 36 est raccordé à la canalisation principale 4 entre le débitmètre 5 et la pompe 6. La différence entre les circuits des figures 1 et 5 réside donc en ce que, dans le circuit de la figure 5, la vanne 10 est disposée en amont de l'électrovanne 7 et en ce que le conduit 36 est raccordé à la conduite principale 4.

Dans le circuit de la figure 5, dans les conditions normales de fonctionnement, le liquide de dialyse circule dans le conduit 36 puis dans le conduit 35 sans qu'il n'y ait passage de liquide du conduit 35 vers le conduit 36 à l'intérieur de la vanne 10. Lorsque la pression en aval de la pompe 6 atteint la valeur de seuil prédéterminée correspondant à l'ouverture de la pompe, notamment en cas de fonctionnement défectueux de la pompe 6, la vanne de sécurité 10 s'ouvre et une partie du liquide circulant dans le conduit 35 se décharge dans le conduit 36. Il en résulte une baisse de pression étant donné que le conduit 36 est relié à l'entrée d'aspiration de la pompe 6. De la sorte, même dans ce circuit, il se produit une baisse de pression dans la conduite principale 4 en aval du conduit 35, ce qui élimine le risques de fuite dans le circuit dus à une désolidarisation partielle des conduites. En outre, même dans ce circuit, il est possible d'effectuer un essai d'étanchéité à la pression pour vérifier le fonctionnement correct de la vanne de sécurité 10. Par ailleurs, dans la mesure où du liquide circule en permanence dans les conduits 35 et 36, il ne se produit pas de stagnation de liquide dans la vanne de sécurité 10.

L'invention n'est pas limitée aux modes de réalisation décrits et représentés, et elle est susceptible de variantes selon des revendications.

## Revendications

1. Circuit hydraulique (1) pour liquide de dialyse comprenant une première section de conduite (4) et une deuxième section de conduite (4; 30) reliées par des moyens de dérivation (10) pour dériver une partie du liquide de dialyse circulant dans la première section de conduite (4) vers la deuxième section de conduite (4; 30) quand la pression dans la première section de conduite (4). dépasse une valeur de seuil prédéterminée, les moyens de dérivation comprenant une vanne (10) comportant un premier conduit (35) et un deuxième conduit (36), **caractérisé en ce que**:
• le premier conduit (35) a une entrée et une sortie raccordées à la première section de conduite (4).
• le deuxième conduit (36) a une entrée et une sortie raccordées à la deuxième section de conduite (4, 30), premier conduit (35) et le deuxième conduit (36) pouvant être en communication de fluide par le biais d'un orifice (37), et
• des moyens d'obturation (39) montés élastiquement pour fermer l'orifice lorsque la pression de liquide dans le premier conduit (35) est inférieure à la valeur de seuil prédéterminée ou pour ouvrir l'orifice lorsque la pression de liquide dans le premier conduit (35) est supérieure à la valeur de seuil prédéterminée.

2. Circuit selon la revendication 1, **caractérisé en ce que** la vanne (10) comprend un corps de vanne (33) ayant une chambre interne tronconique (38) à laquelle les deux conduits (35, 36) sont raccordés de façon à être superposés.

3. Circuit selon la revendication 2, **caractérisé en ce que** les deux conduits (35, 36) communiquent par l'intermédiaire de la chambre tronconique (38), le conduit supérieur (35) ayant deux tronçons diamétralement opposés débouchant dans la paroi latérale de la chambre tronconique (38) et la chambre tronconique (38) ouvrant à son extrémité de plus petit diamètre, par l'orifice (37), dans le conduit inférieur (35).

4. Circuit selon la revendication 3, **caractérisé en ce que** les moyens d'obturation comprennent un obturateur (39) logé dans la chambre tronconique (38), l'obturateur (39) ayant une extrémité arrondie dimensionnée pour s'engager partiellement dans l'orifice (37) et étant maintenu plaqué élastiquement sur le pourtour de l'orifice (38) par un ressort (41) prenant appui sur une paroi du corps de vanne (33).

5. Circuit selon l'une des revendications 1 à 4, **caractérisé en ce que** la première section de conduite fait partie d'une conduite principale (4) et **en ce que** la deuxième section de conduite fait partie d'une conduite auxiliaire (30) connectée à la conduite principale (4) au moyen d'une vanne trois voie (7).

6. Circuit selon la revendication 5, **caractérisé en ce que** le premier conduit (35) de la vanne (10) est raccordé à la conduite principale (4) en aval de la vanne trois voies (7), et **en ce que** le deuxième conduit (36) de la vanne (10) est raccordé à la conduite auxiliaire (30) en aval de la vanne trois voies (7).

7. Circuit selon la revendication 5, **caractérisé en ce que** le premier conduit (35) de la vanne (10) est raccordé à la conduite principale (4) en amont de la vanne trois voies (7), et **en ce que** le deuxième conduit (36) de la vanne (10) est raccordé à la conduite auxiliaire (30) en aval de la vanne trois voie (7).

8. Circuit selon l'une des revendications 1 à 4, **caractérisé en ce que** la première section de conduite fait partie d'une conduite principale (4) munie d'une pompe (6), **en ce que** le premier conduit (35) de la vanne (10) est raccordé à la conduite principale (4) en aval de la pompe (6), et **en ce que** le deuxième conduit (36) de la vanne (10) est raccordé à la conduite auxiliaire (30) en amont de la pompe (6).

## Claims

1. Hydraulic circuit (1) for dialysis liquid comprising a first section of pipe (4) and a second section of pipe (4; 30) which sections are connected by diversion means (10) to divert some of the dialysis liquid flowing through the first section of pipe (4) to the second section of pipe (4; 30) when the pressure in the first section of pipe (4) exceeds a predetermined threshold value, the diversion means comprising a valve (10) comprising a first duct (35) and a second duct (36), **characterized in that**:
• the first duct (35) has an inlet and an outlet that are connected to the first section of pipe (4),
• the second duct (36) has an inlet and an outlet that are connected to the second section of pipe (4; 30), the first duct (35) and the second duct (36) being able to be in fluidic communication via an orifice (37), and
• shut-off means (39) mounted elastically to close the orifice when the liquid pressure in the first duct (35) is lower than the predetermined threshold value or to open the orifice when the liquid pressure in the first duct (35) is higher than the predetermined threshold value.

2. Circuit according to Claim 1, **characterized in that** the valve (10) comprises a valve body (33) having a frustoconical internal chamber (38) to which the two ducts (35, 36) are connected in such a way as to be superposed.

3. Circuit according to Claim 2, **characterized in that** the two ducts (35, 36) communicate via the frustoconical chamber (38), the upper duct (35) having two diametrically opposed portions opening into the side wall of the frustoconical chamber (38) and the frustoconical chamber (38) opening at its smaller-diameter end via the orifice (37) into the lower duct (35).

4. Circuit according to Claim 3, **characterized in that** the shut-off means comprise a shutter (39) housed in the frustoconical chamber (38), the shutter (39) having a rounded end sized to fit partially into the orifice (37) and being held pressed elastically against the periphery of the orifice (38) via a spring (41) bearing against a wall of the valve body (33).

5. Circuit according to one of Claims 1 to 4, **characterized in that** the first section of pipe forms part of a main pipe (4) and **in that** the second section of pipe forms part of an auxiliary pipe (30) connected to the main pipe (4) by means of a three-way valve (7).

6. Circuit according to Claim 5, **characterized in that** the first duct (35) of the valve (10) is connected to the main pipe (4) downstream of the three-way valve (7), and **in that** the second duct (36) of the valve (10) is connected to the auxiliary pipe (30) downstream of the three-way valve (7).

7. Circuit according to Claim 5, **characterized in that** the first duct (35) of the valve (10) is connected to the main pipe (4) upstream of the three-way valve (7), and **in that** the second duct (36) of the valve (10) is connected to the auxiliary pipe (30) downstream of the three-way valve (7).

8. Circuit according to one of Claims 1 to 4, **characterized in that** the first section of pipe forms part of a main pipe (4) fitted with a pump (6), **in that** the first duct (35) of the valve (10) is connected to the main pipe (4) downstream of the pump (6), and **in that** the second duct (36) of the valve (10) is connected to the auxiliary pipe (30) upstream of the pump (6).

## Patentansprüche

1. Hydraulikkreis (1) für Dialyseflüssigkeit mit einem ersten Leitungsabschnitt (4) und einem zweiten Leitungsabschnitt (4; 30), die durch Verzweigungsmittel (10) zum verbunden sind, um einen Teil der in dem ersten Leitungsabschnitt (4) zirkulierenden Dialyseflüssigkeit zu dem zweiten Leitungsabschnitt (4; 30) abzuzweigen, wenn der Druck in dem ersten Leitungsabschnitt (4) einen vorbestimmten Grenzwert überschreitet, wobei die Verzweigungsmittel ein Ventil (10) umfassen, das eine erste Leitung (35) und eine zweite Leitung (36) umfasst, **dadurch gekennzeichnet, dass**
- die erste Leitung (35) einen Eingang und einen Ausgang aufweist, die an den ersten Leitungsabschnitt (4) angeschlossen sind,
- die zweite Leitung (36) einen Eingang und einen Ausgang aufweist, die an den zweiten Leitungsabschnitt (4; 30) angeschlossen sind, wobei die erste Leitung (35) und die zweite Leitung (36) auf dem Umweg über eine Öffnung (37) in Fluidverbindung stehen können, und
- Verschlussmittel (39), die elastisch angebracht sind, um die Öffnung zu schließen, wenn der Flüssigkeitsdruck in der ersten Leitung (35) kleiner als der vorbestimmte Grenzwert ist, oder um die Öffnung zu öffnen, wenn der Flüssigkeitsdruck in der ersten Leitung (35) größer als der vorbestimmte Grenzwert ist.

2. Kreislauf nach Anspruch 1, **dadurch gekennzeichnet, dass** das Ventil (10) einen Ventilkörper (33) mit einer kegelstumpfförmigen Innenkammer (38) umfasst, an die die zwei Leitungen (35, 36) so angeschlossen sind, dass sie übereinander liegen.

3. Kreislauf nach Anspruch 2, **dadurch gekennzeichnet, dass** die zwei Leitungen (35, 36) mittels der kegelstumpfförmigen Kammer (38) in Verbindung stehen, wobei die obere Leitung (35) zwei diametral einer Hauptleitung (4) ist, die mit einer Pumpe (6) versehen ist, dass die erste Leitung (35) des Ventils (10) ans die Hauptleitung (4) stromabwärts von der Pumpe (6) angeschlossen ist und dass die zweite Leitung (36) des Ventils (10) stromaufwärts von der Pumpe (6) an die Hilfsleitung (30) angeschlossen ist. gegenüberliegende Abschnitte aufweist, die in der Seitenwand der kegelstumpfförmigen Kammer (38) münden, und die kegelstumpfförmigen Kammer (38) sich an ihrem Ende mit dem kleineren Durchmesser über die Öffnung (37) in die untere Leitung (35) öffnet.

4. Kreislauf nach Anspruch 3, **dadurch gekennzeichnet, dass** die Verschlussmittel einen Verschluss (39) umfassen, der in der kegelstumpfförmigen Kammer (38) untergebracht ist, wobei der Verschluss (39) ein abgerundetes Ende aufweist, das so bemessen ist, dass es teilweise in die Öffnung (37) eingreift, und das wird durch eine Feder (41), die an einer Wand des Ventilkörpers (33) anliegt, elastisch auf den Umfang der Öffnung (38) gepresst gehalten wird.

5. Kreislauf nach einem der Ansprüche 1 bis 4, **dadurch gekennzeichnet, dass** der erste Leitungsabschnitt Teil einer Hauptleitung (4) ist und dass der zweite Leitungsabschnitt Teil einer Hilfsleitung (30) ist, die an die Hauptleitung (4) mittels einen Dreiwegeventils (7) angeschlossen ist.

6. Kreislauf nach Anspruch 5, **dadurch gekennzeichnet, dass** die erste Leitung (35) des Ventils (10) an die Hauptleitung (4) stromabwärts von dem Dreiwegeventil (7) angeschlossen ist und dass die zweite Leitung (36) des Ventils (10) stromabwärts von dem Dreiwegeventil (7) an die Hilfsleitung (30) angeschlossen ist.

7. Kreislauf nach Anspruch 5, **dadurch gekennzeichnet, dass** die erste Leitung (35) des Ventils (10) an die Hauptleitung (4) stromaufwärts von dem Dreiwegeventil (7) angeschlossen ist und dass die zweite Leitung (36) des Ventils (10) stromabwärts von dem Dreiwegeventil (7) an die Hilfsleitung (30) angeschlossen ist.

8. Kreislauf nach einem der Ansprüche 1 bis 4, **dadurch gekennzeichnet, dass** der erste Leitungsabschnitt Teil
